(19) Europäisches Patentamt
European Patent Office
Office européen des brevets

(11) **EP 2 781 189 B1**

(12) **EUROPEAN PATENT SPECIFICATION**

(45) Date of publication and mention
of the grant of the patent:
**18.04.2018 Bulletin 2018/16**

(51) Int Cl.:
*A61B 5/0492* *(2006.01)*    *A61B 5/0488* *(2006.01)*
*A61B 5/11* *(2006.01)*    *A61B 5/00* *(2006.01)*

(21) Application number: **14155345.3**

(22) Date of filing: **17.02.2014**

(54) **State determination device, state determination method, and state determination program**

Zustandsbestimmungsvorrichtung, Zustandsbestimmungsverfahren und
Zustandsbestimmungsprogramm

Dispositif, procédé et programme de détermination d'état

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.03.2013 JP 2013055114**

(43) Date of publication of application:
**24.09.2014 Bulletin 2014/39**

(73) Proprietor: **FUJITSU LIMITED
Kawasaki-shi,
Kanagawa 211-8588 (JP)**

(72) Inventor: **Kasama, Kouichirou
Kawasaki-shi, Kanagawa 211-8588 (JP)**

(74) Representative: **Ward, James Norman et al
Haseltine Lake LLP
Bürkleinstrasse 10
80538 München (DE)**

(56) References cited:
**US-A1- 2011 118 621    US-A1- 2012 184 871**

- **"Signal characteristics of EMG during fatigue -
Springer", , 16 September 1977 (1977-09-16),
XP055115324, Retrieved from the Internet:
URL:http://rd.springer.com/article/10.1007
/BF00421697#page-1 [retrieved on 2014-04-25]**
- **HOWARD J GREEN ET AL: "Adaptations in
muscle metabolic regulation require only a small
dose of aerobic-based exercise", EUROPEAN
JOURNAL OF APPLIED PHYSIOLOGY,
SPRINGER, BERLIN, DE, vol. 113, no. 2, 17 June
2012 (2012-06-17) , pages 313-324, XP035164020,
ISSN: 1439-6327, DOI:
10.1007/S00421-012-2434-5**

**Description**

FIELD

The embodiments discussed herein are related to a state determination device, a state determination method, and a state determination program, which determine a state of the body.

BACKGROUND

Methods for determining presence/absence of fatigue of the body or an aerobic exercise are conventionally known. For example, fatigue or an aerobic exercise can be determined by analyzing components of blood. In this case, however, it is needed to draw blood from the body and analyze the blood by a dedicated research institute. This analysis sometimes needs several days, posing problems such that a physical and financial burden is imposed to determine fatigue or an aerobic exercise, and it is difficult to measure fatigue or an aerobic exercise in real time.

Related to the above described techniques, a method for obtaining an anaerobic threshold of a user by measuring heartbeats of the user when he or she does a physical exercise while changing an exercise load gradually or in stages, and by analyzing results of the measurement with a specified method is known.

Additionally, a muscle fatigue evaluation device for calculating an integrated value by processing, as absolute values, a time series signal having a gradient of a power value obtained by performing a specified operation for signal data obtained from a myopotential detection sensor, and a time series signal having a gradient of the largest Lyapunov exponent, and for using the integrated value as the degree of fatigue (the progress of fatigue) is known.

Furthermore, an exercise machine for estimating an anaerobic threshold based on an electro-cardiographic signal in response to a load change of a load device, and for controlling a load based on the estimated value is known.

Documents of Prior Art

Patent Documents

Patent Document 1: Japanese Laid-open Patent Publication No. 2007-075172
Patent Document 2: Japanese Laid-open Patent Publication No. 2007-209453
Patent Document 3: National Publication of International Patent Application No. WO/1999/043392

US 2011/0118621 discloses a muscular energy state analysis system and method for swing motion.

SUMMARY

An object in an aspect of the embodiments is to provide a state determination device that can simply determine a state of the body.

According to an aspect of the embodiments, a state determination device includes the following components.

A myopotential measurement unit measures a myopotential of a surface of the body.

A myopotential integrated value calculation unit calculates a myopotential integrated value by integrating, with time, the myopotential measured by the myopotential measurement unit for a measurement period at predetermined measurement intervals.

A fatigue determination unit determines presence/absence of fatigue of the body based on a size of the myopotential integrated value calculated by the myopotential integrated value calculation unit, and a change amount of the myopotential integrated value.

BRIEF DESCRIPTION OF DRAWINGS

FIG. 1 is an explanatory diagram of a state determination device according to an embodiment;
FIG. 2 illustrates a configuration example of a portable terminal device for implementing the state determination device, according to another embodiment;
FIG. 3 is an external view of the portable terminal device;
FIG. 4 is a flowchart illustrating a state determination process executed by the portable terminal device according to the embodiment;
FIG. 5 is a flowchart illustrating the state determination process executed by the portable terminal device according to the embodiment;
FIG. 6 illustrates time characteristics of a myopotential integrated value at the time of an aerobic exercise;
FIG. 7 illustrates a relationship between an acceleration and an exercise intensity;
FIG. 8 illustrates a display example of process results in steps S410 to S413;
FIG. 9 illustrates a display example of process results in steps S414 to S417;
FIG. 10 illustrates another configuration example of a myopotential sensor;
FIG. 11 illustrates a further configuration example of the myopotential sensor; and
FIG. 12 illustrates a configuration of an information processing device.

DESCRIPTION OF EMBODIMENTS

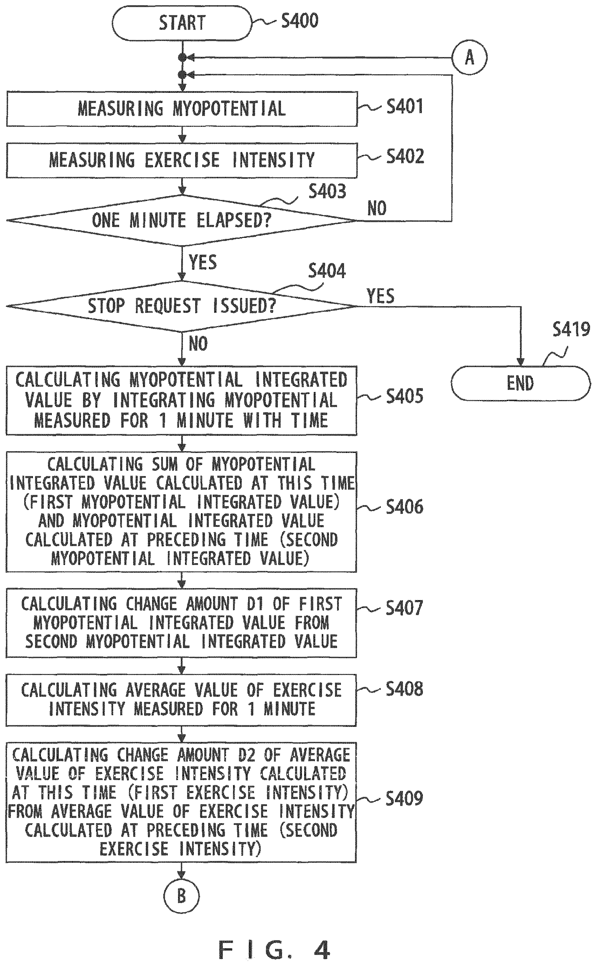

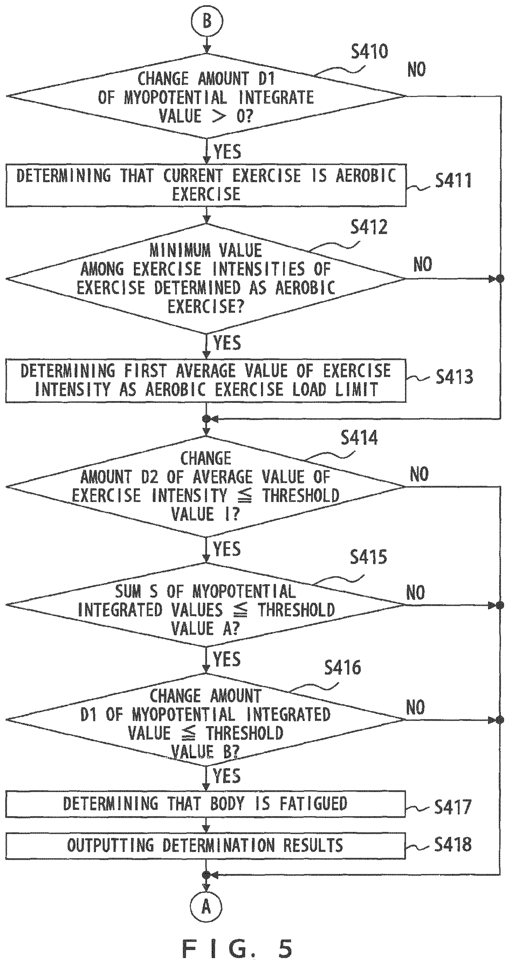

Embodiments are described in detail below with reference to FIGs . 1 to 11. The following embodiments are merely examples, and are not intended to preclude various modifications and technical applications, which are not explicitly described below. Namely, the embodiments may be variously modified and implemented, for example, by combining techniques recited as embodiments within a scope that does not depart from the gist of the embodiments. Moreover, process procedures represented by the flowcharts in FIGs. 4 and 5 are not intended to limit the order of processes. Accordingly, the order of processes may be naturally changed if possible.

<Embodiment>

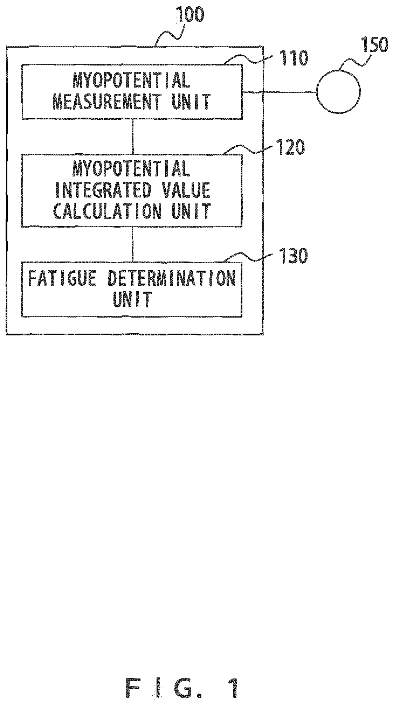

FIG. 1 is an explanatory diagram of a state determination device 100 according to an embodiment.
The state determination device 100 includes a myopotential measurement unit 110, a myopotential integrated value calculation unit 120, and a fatigue determination unit 130. Note that the myopotential integrated value calculation unit 120 and the fatigue determination unit 130 may be implemented by causing a CPU, which is included in the state determination device 100 and not illustrated, to execute a specified program.
The myopotential measurement unit 110 measures a myopotential of a surface of the body 150. As the myopotential measurement unit 110, a myopotential sensor or the like is available.
The myopotential integrated value calculation unit 120 calculates a myopotential integrated value by integrating, with time, the myopotential measured by the myopotential measurement unit 110 for a measurement period at predetermined measurement intervals.
The fatigue determination unit 130 determines presence/absence of fatigue of the body 150 based on a size of the myopotential integrated value calculated by the myopotential integrated value calculation unit 120 and a change amount of the myopotential integrated value. For example, the fatigue determination unit 130 calculates the change amount of the myopotential integrated value calculated by the myopotential integrated value calculation unit 120 at this time from the myopotential integrated value calculated by the myopotential integrated value calculation unit 120 at preceding time. Then, the fatigue determination unit 130 can determine that the body 150 is fatigued if the myopotential integrated value is equal to or smaller than a threshold value X and the calculated change amount of the myopotential integrated value is equal to or smaller than a threshold value Y. This adopts the phenomenon that myopotential actions are reduced when the body 150 is getting fatigued, leading to a decrease in the value of a myopotential. Note that a predetermined positive real value is available as the threshold value X. Moreover, a predetermined real value is available as the threshold value Y.
As described above, the state determination device 100 determines the presence/absence of the fatigue of the body 150 based on the size of the myopotential integrated value calculated by integrating, with time, the myopotential measured from the body 150 for a measurement period, and the change amount of the myopotential integrated value. Accordingly, with the state determination device 100, a state of the body can be easily determined without performing operations such as analyzing components of blood after drawing the blood from the body 150.

<Another embodiment>

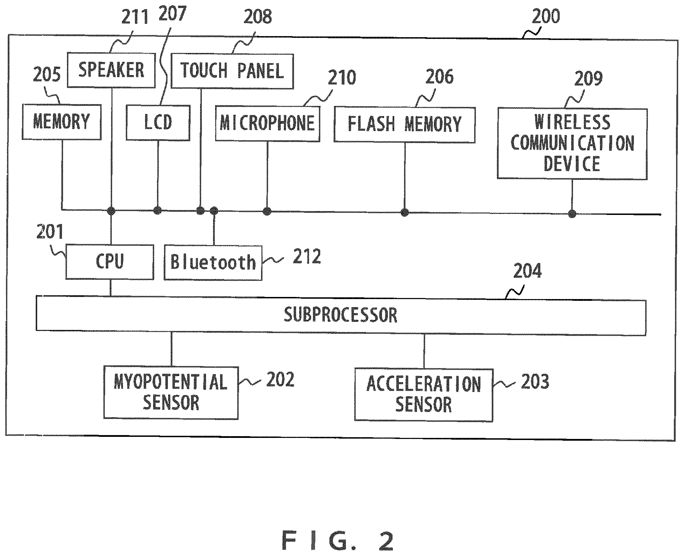

FIG. 2 illustrates a configuration example of a portable terminal device 200 that implements the state determination device, according to another embodiment.
The portable terminal device 200 includes a CPU (Central Processing Unit) 201, a myopotential sensor 202, an acceleration sensor 203, a subprocessor 204, a memory 205, a flash memory 206, an LCD (Liquid Crystal Display) 207, and a touch panel 208.
The CPU 201 is an arithmetic processing unit for executing a program for implementing a state determination process according to this embodiment in addition to execution of peripheral devices and various types of software. For example, the CPU 201 executes the state determination process illustrated in FIGs. 4 and 5 according to a program expanded in the memory 205.

The CPU 201 calculates a myopotential integrated value by integrating, with time, a myopotential measured by the myopotential sensor 202 per unit time, for one minute in this embodiment. Then, the CPU 201 determines whether or not the body of a user of the portable terminal device 200 is doing an aerobic exercise based on the myopotential integrated value per unit time and a change of the value.

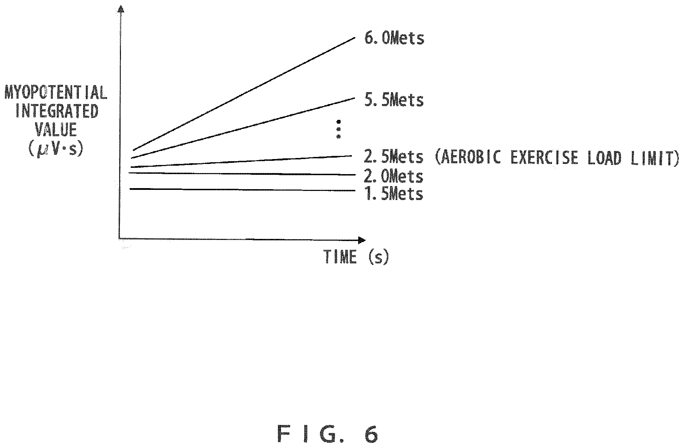

This embodiment can adopt the phenomenon that a myopotential increases with time if an aerobic exercise is continued with a certain exercise intensity. For instance, in the example of FIG. 6, a myopotential integrated value is constant when an exercise is done with an exercise intensity of 1.5 [Mets] or 2.0 [Mets]. In contrast, when an exercise is done with an exercise intensity of 2.5 [Mets] or higher, the myopotential integrated value increases. As described above, by detecting an increase in the myopotential integrated value when an exercise is done with a certain exercise intensity, the CPU 201 can determine that the body of a user of the portable terminal device 200 is doing an aerobic exercise. In this embodiment, the CPU 201 determines that the body of the user of the portable terminal device 200 is doing an aerobic exercise when a change amount D1 of the myopotential integrated value, which will be described later, is larger than 0.

In the example of FIG. 6, it is proved that the user of the portable terminal device 200 can do an aerobic exercise by doing an exercise with an exercise intensity of 2.0 [Mets] or higher, namely, 2.5 [Mets]. A boundary of the exercise intensity, at which an aerobic exercise can be done in this way, is referred to as an "aerobic exercise load limit". The CPU 201 can estimate the aerobic exercise load limit based on an exercise intensity of an exercise when the exercise is determined to be an aerobic exercise at present or in the past.

Additionally, the CPU 201 calculates an average value of an exercise intensity per unit time, which is calculated by the subprocessor 204, as will be described later. Then, the CPU 201 determines the presence/absence of the fatigue of the body based on largeness/smallness of the myopotential integrated value, and a change amount of the myopotential integrated value if the change amount D2 of the exercise intensity, which will be described later, is within a certain range, equal to or smaller than a threshold value I in this embodiment. As the threshold value I, a positive real number is available.

The reason why the change amount D2 of the exercise intensity is limited to the certain range is to exclude a case where an exercise intensity suddenly changes. Accordingly, the threshold value I can be set based on, for example, an allowable maximum change amount of an exercise intensity. "fatigue of the body" in this embodiment means fatigue of muscles, and can include a state where lactic acid starts to accumulate in muscles due to muscle actions.

Additionally, when the body is getting fatiguing, an amplitude of a potential of muscle actions decreases and an increment of the myopotential integrated value becomes small or reduces even though an exercise is done with the same exercise intensity. Therefore, the CPU 201 according to this embodiment determines that the body is fatigued when a sum S of the myopotential integrated value, which will be described later, is equal to or smaller than a threshold value A and the change amount D1 of the myopotential integrated value is equal to or smaller than a threshold value B.

A predetermined positive real value is available as the threshold value A. Moreover, a predetermined real value is available as the threshold value B. If the change amount D1 of the myopotential integrated value indicates a negative real value, this means that the myopotential integrated value decreases with time. Accordingly, if the change amount D of the myopotential integrated value indicates a negative real value, which is extremely smaller than 0, the user of the portable terminal device 200 has started to feel fatigued in some cases. Accordingly, by setting a positive real value or negative real value close to 0 as the threshold value B, the portable terminal device 200 can detect the fatigue of the body before the user of the portable terminal device 200 starts to feel fatigued.

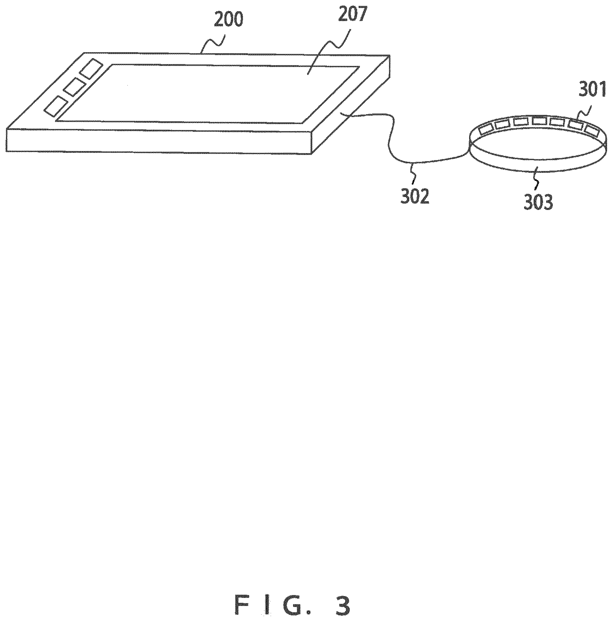

The myopotential sensor 202 can include electrodes 301 connected via a signal line 302 as illustrated in FIG. 3 to be described later although they are not illustrated in FIG. 2. The myopotential sensor 202 outputs a myopotential of a surface of the body, which makes contact with the electrodes 301, to the subprocessor 204 via the electrodes 301.

The acceleration sensor 203 is a device for measuring an acceleration of the portable terminal device 200 mounted on the body, namely, an acceleration applied to the body. The subprocessor 204 calculates an exercise intensity and an action quantity by using the acceleration measured by the acceleration sensor 203 as will be described later.

The subprocessor 204 notifies the CPU 201 of a myopotential after the subprocessor 204 performs a specified operation such as converting a myopotential output by the myopotential sensor 202 into digital data. Moreover, the subprocessor 204 notifies the CPU 201 of calculation results after the subprocessor 204 calculates an exercise intensity and an action quantity based on the acceleration in a particular direction, for example, the gravity direction, which is measured by the acceleration sensor 203.

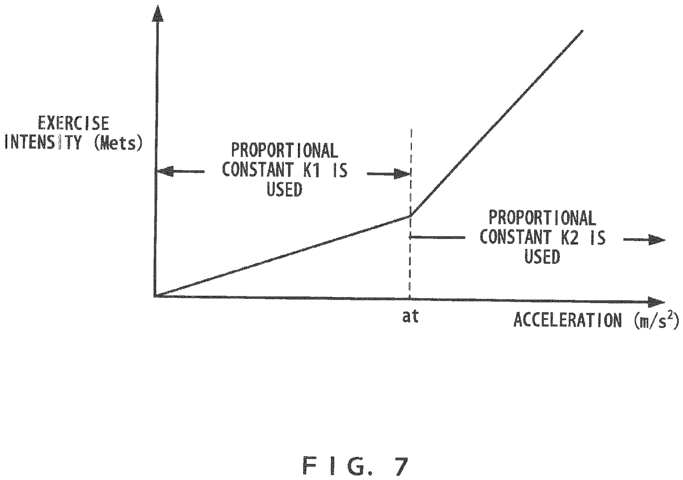

In this embodiment, the subprocessor 204 can obtain the exercise intensity by using a relational expression, illustrated in FIG. 7, between an acceleration and an exercise intensity as follows. The relational expression illustrated in FIG. 7 can be represented with the following equations.

$$(\text{exercise intensity [Mets]}) = k1 \times (\text{acceleration a1}),$$
$$0 \leq a1 < at \ldots (1)$$

$$(\text{exercise intensity [Mets]}) = k2 \times (\text{acceleration a2}),$$
$$at \leq a2 \ldots (2)$$

Proportional constants k2, k2, a1, a2, and at are predetermined real values. These constants can be obtained by statistically approximating a relationship between an exercise intensity and an acceleration in a particular direction, for example, the gravity direction, which is applied to the body at the time of the exercise with the exercise intensity when the exercise, such as running, is done with a plurality of exercise intensities.

The subprocessor 204 calculates an action quantity at a time $\Delta t$[s] with the following expression (3) after the subprocessor 204 calculates the exercise intensity based on the acceleration measured by the acceleration sensor 203 with the use of the expressions (1) and (2).

$$(\text{action quantity [Mets·h]}) = (\text{exercise intensity [Mets]})$$
$$\times \Delta t \div (60 \times 60) \ldots (3)$$

The exercise intensity calculation method according to this embodiment is not limited to the above described one as a matter of course. For example, an exercise intensity corresponding to an acceleration obtained from the acceleration sensor 203 may be stored in the memory 205 as a table. Alternatively, an exercise intensity may be calculated by using information other than an acceleration.

The memory 205 is a volatile storage device for temporarily storing a program and data, which the portable terminal device 200 needs to operate, and part or the whole of a program for implementing the state determination process according to this embodiment. As the memory 205, for example, a RAM (Random Access Memory) or the like is available.

The flash memory 206 is a nonvolatile storage device for storing the program for implementing the state determination process according to this embodiment in addition to the program and the data, which the portable terminal device 200 needs to operate.

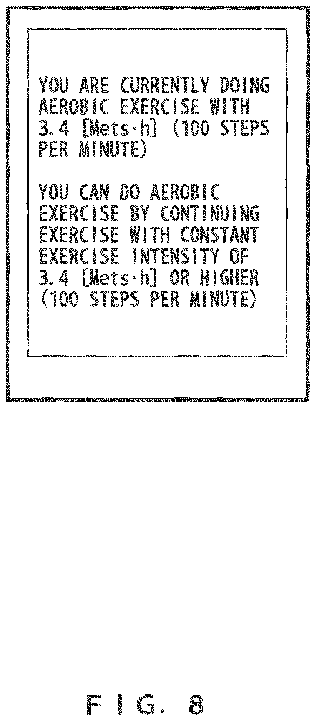

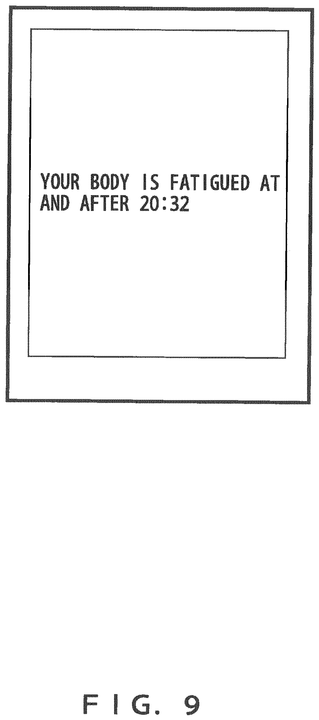

The LCD 207 is a display device for displaying data and the like output by the CPU 201 or the like, for example, data illustrated in FIGs. 8 and 9. The touch panel 208 is data input means from an outside. For example, upon detection of a specified operation that a user performs on the touch panel 208, the CPU 201 starts the state determination process according to this embodiment.

Additionally, the portable terminal device 200 can include a wireless communication device 209, a microphone 210, a speaker 211, and Bluetooth (registered trademark) 212.

The wireless communication device 209 is, for example, a communication device for communicating with another portable terminal device by making a wireless communication with a base station that connects to a specified communication network. For example, the wireless communication device 209 outputs, to the speaker 211, voice data generated by executing processes for transmitting, to the other portable terminal device, voice data of a voice input to the microphone 210, and for decoding the signal received from the other portable terminal device.

The microphone 210 is a device for outputting an input voice or the like as a voice signal. Moreover, the speaker 211 is a reproduction device for reproducing a voice or the like from voice data which is received from another portable terminal device and for which processes such as decoding and the like are executed.

As a storage medium readable by the portable terminal device 200, such as the memory 205, the flash memory 206 or the like, a non-transitory medium is available.

Additionally, FIG. 2 depicts the configuration example in the case where the state determination device according to this embodiment is implemented with the portable terminal device 200 such as a cellular phone or the like. This is merely one example. Accordingly, to implement the state determination device according to this embodiment, unneeded components may be suitably removed from among the components illustrated in FIG. 2, or a new component, not illustrated in FIG. 2, may be added.

FIG. 3 is an external view of the portable terminal device 200. In the portable terminal device 200, the signal line 302 connecting to the myopotential sensor 200 within the portable terminal device 200 is connected to the electrodes 301 as illustrated in FIG. 3. The plurality of electrodes 301 are attached to an inside of a band 303 made of an elastic material. The band 303 is attached to an arm or the like of a user to make contact with a skin surface of the user, so that the myopotential sensor 202 can measure a myopotential via the electrodes 301 in contact with the skin surface of the user.

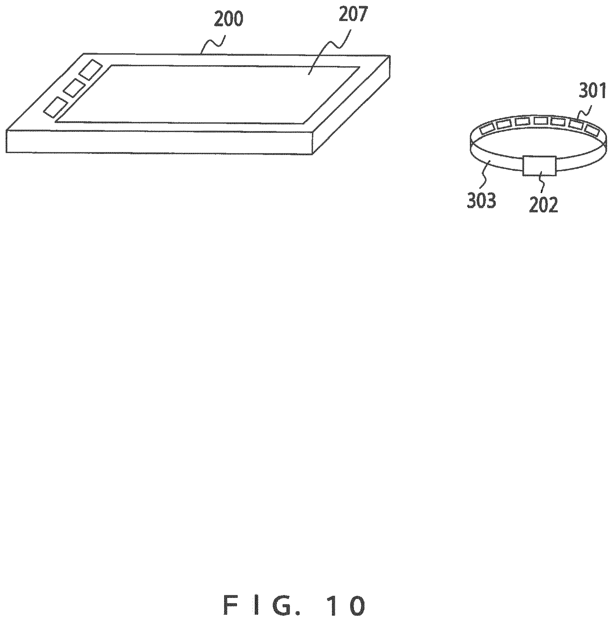

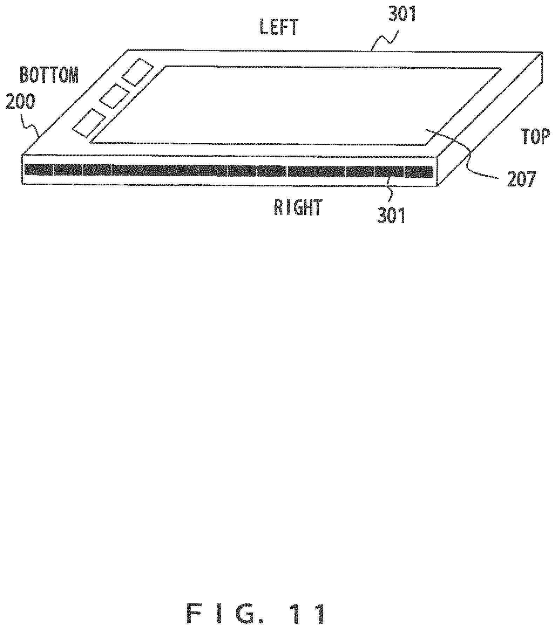

The electrodes 301, the signal line 302, and the band 303, which are illustrated in FIG. 3, are merely examples used in this embodiment. Accordingly, the arrangement and the shape of the electrodes 301, and the connection with the myopotential sensor 202 are not limited to those illustrated in FIG. 3. For example, the myopotential sensor 202 may be attached to the band 303 as illustrated in FIG. 10. In this case, by attaching a Bluetooth transmitter to the band 303 along with the myopotential sensor 202, the Bluetooth transmitter can transmit the myopotential that the myopotential sensor 202 measures via the electrodes 301 to the portable terminal device 200 . Moreover, the electrodes 301 may be provided on a right side surface and a left side surface of the portable terminal device 200 as illustrated in FIG. 11.

FIGs. 4 and 5 are flowcharts illustrating the state determination process of the body, which is executed by the portable terminal device 200 according to this embodiment.

Upon detection of a specified operation of a user on the touch panel 208, the portable terminal device 200 starts the state determination process (step S401).

The portable terminal device 200 measures a myopotential and an exercise intensity for one minute at intervals of one second (steps S401 and S402, "NO" in step S403). The portable terminal device 200 can obtain the myopotential from the myopotential sensor 202. Moreover, the portable terminal device 200 can obtain, from the subprocessor 204, an exercise intensity calculated based on an acceleration obtained from the acceleration sensor 203 as described above with reference to FIG. 2.

After the portable terminal device 200 measures the myopotential and the exercise intensity for one minute ("YES" in S403), the portable terminal device 200 shifts the process to step S404. If a stop request of the state determination process is issued with a specified operation of the user ("YES" in step S404), the portable terminal device 200 terminates the state determination process (step S419).

If the stop request of the state determination process is not issued ("NO" in step S404), the portable terminal device 200 calculates a myopotential integrated value ($\mu$V·s) by integrating the myopotential measured for one minute in step S401 with time (step S405) . Then, the portable terminal device 200 calculates a sum S of the myopotential integrated value calculated at this time (hereinafter referred to as a first myopotential integrated value) in step S405 and a myopotential integrated value calculated at the preceding time (hereinafter referred to as a second myopotential integrated value) in step S405 (step S406).

Adopting the sum S in this embedment is to improve accuracy of the determination of the presence/absence of fatigue, which uses the myopotential integrated value, by reducing an extreme influence on a value of the myopotential with the use of the myopotential integrated value obtained for the longest possible period. Accordingly, not only the sum of the myopotential integrated value calculated at this time and that calculated at the preceding time but a sum of the myopotential integrated value calculated at this time, that calculated at the preceding time, and that calculated at time antecedent to the preceding time in step S405 is available.

Additionally, the portable terminal device 200 calculates the change amount D1 of the first myopotential integrated value from the second myopotential integrated value (step S407).

Furthermore, the portable terminal device 200 calculates an average value of the exercise intensity measured for one minute in step S402 (step S408). Then, the portable terminal device 200 calculates the change amount D2 of the average value of the exercise intensity, which is hereinafter referred to as a first exercise intensity and calculated at this time in step S408, from an average value of the exercise intensity, which is hereinafter referred to as a second exercise intensity and calculated at the preceding time in step S408 (step S409).

If the change amount D1 of the myopotential integrated value calculated in step S407 is larger than 0 in step S410 ("YES" in step S410), the portable terminal device 200 determines that the current exercise is an aerobic exercise (step S411) . Then, the portable terminal device 200 stores the average value of the exercise intensity calculated in step S408, namely, the first exercise intensity at a specified address of the flash memory 206.

Additionally, if the first exercise intensity obtained when the exercise is determined as the aerobic exercise in step S411 is a minimum value among average values of the exercise intensity when the exercise is determined as the aerobic exercise ("YES" in step S412), the portable terminal device 200 shifts the process to step S413. In this case, the portable terminal device 200 estimates the average value of the exercise intensity of the exercise when the exercise is determined as the aerobic exercise in step S411, namely, the first exercise intensity as an aerobic exercise load limit (step S413). Accuracy of this estimation can be improved by repeating the process of step S413, namely, by user doing an exercise with diverse exercise intensities with the use of the portable terminal device 200.

If the change amount D2 of the exercise intensity, which is calculated in step S409, is equal to or lower than the threshold value I in step S414 ("YES" in step S414), the portable terminal device 200 shifts the process to step S415. In this case, the sum S of the myopotential integrated values, which is calculated in step S406, is equal to or smaller than the threshold value A ("YES" in step S415) and the change amount D1 of the myopotential integrated value, which is calculated in step S407, is equal to or smaller than the threshold value B ("YES" in step S416), the portable terminal device 200 determines that the body is fatigued (step S417). Then, the portable terminal device 20 displays determination results of steps S410 to S417 on a display screen of the portable terminal device 200 (step S418).

For example, the portable terminal device 200 can notify a user of the process results in steps S410 to S413 by displaying a display screen illustrated in FIG. 8. In the example of FIG. 8, a user is notified of an aerobic exercise currently being done, the current action quantity, and the aerobic exercise load limit. As the current action quantity, an action quantity (Mets·h) obtained by using the expression (3) based on the exercise intensity calculated in step S402 is available. Moreover, as the aerobic exercise load limit, an action quantity (Mets·h) obtained by using the expression (3) based on the first exercise intensity estimated as the aerobic exercise load limit in step S413 is available.

Additionally, for example, the portable terminal device 200 can notify a user of the process results in steps S414 to S417 by displaying a display screen illustrated in FIG. 9. In the example of FIG. 9, it is notified to a user that his or her body is fatigued.

The displays illustrated in FIGs. 8 and 9 may be displayed on one display screen as a matter of course. Moreover, in the flowchart of FIG. 5, the portable terminal device 200 displays the determination results of steps S410 to S417 in step S418 only if the body is determined to be fatigued. However, the portable terminal device 200 may display the process results of steps S410 to S413, for example, each time the process of step S413 is executed.

As described above, the portable terminal device 200 measures a myopotential from a skin surface of the body. Then, the portable terminal device 200 calculates a myopotential integrated value based on the myopotential measured per unit time, for every one minute in this embodiment. Then, the portable terminal device 200 determines that the body is fatigued if the sum S of the myopotential integrated values is equal to or smaller than the threshold value A and the change amount D1 of the myopotential integrated value is equal to or smaller than the threshold value B ("YES" in step S415, "YES" in step S416).

In this way, the portable terminal device 200 can determine fatigue of the body based on a myopotential of a skin surface of the body. Accordingly, components of blood do not need to be analyzed in order to determine the fatigue of the body. Accordingly, with the portable terminal device 200, a state of the body can be easily determined.

Additionally, the portable terminal device 200 determines that the body is doing an aerobic exercise if the change amount of the myopotential integrated value calculated per unit time, for every one minute in this embodiment is larger than 0 ("YES" in step S410) . Accordingly, with the portable terminal device 200, whether or not the body is doing an aerobic exercise can be determined without performing special operations such as an analysis of components of blood.

Furthermore, the portable terminal device 200 determines the fatigue of the body based on a myopotential integrated value calculated based on a myopotential measured per unit time and the change amount of the myopotential integrated value, whereby the portable terminal device 200 can determine the fatigue of the body in a short time, for example, two minutes at the shortest in the case where the unit time is one minute. Similarly, the portable terminal device 200 can determine, in a short time, whether or not the body is doing an aerobic exercise.

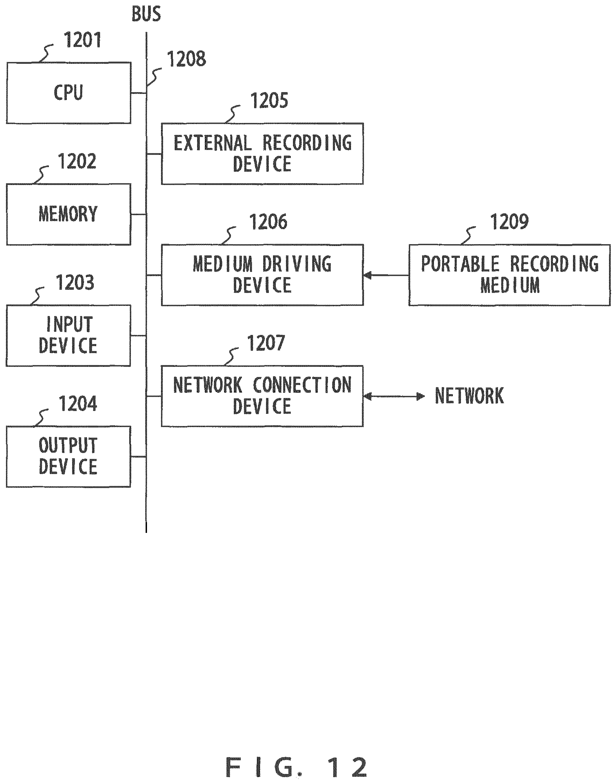

The above described state determination process can be implemented by using, for example, an information processing device (computer) illustrated in FIG. 12. The information processing device illustrated in FIG. 12 includes a CPU (Central Processing Unit) 1201, a memory 1202, an input device 1203, an output device 1204, an external recording device 1205, a medium driving device 1206, and a network connection device 1207. These components are interconnected by a bus 1208.

The memory 1202 is a semiconductor memory such as a ROM (Read Only Memory), a RAM (Random Access Memory), a flash memory, or the like, and stores a program and data, which are used for the state determination process. For example, the CPU 1201 executes the above described state determination process by executing the program with the use of the memory 1202.

The input device 1203 is, for example, a keyboard, a pointing device, or the like, and is used to input an instruction and information from an operator. The output device 1204 is, for example, a display device, a printer, a speaker, or the like, and used to output an inquiry or a process result to an operator.

The external recording device 1205 is, for example, a magnetic disk device, an optical disk device, a magneto-optical disk device, a tape device, or the like. The external recording device 1205 also includes a hard disk drive. The information processing device stores a program and data in the external recording device 1205, and can use the program and the data by loading them into the memory 1202.

The medium driving device 1206 drives a portable recording medium 1209, and accesses recorded contents of the medium. The portable recording medium 1209 is a memory device, a flexible disk, an optical disk, a magneto-optical disk, or the like. The portable recording medium 1209 also includes a Compact Disk Read Only Memory (CD-ROM), a Digital Versatile Disk (DVD), a Universal Serial Bus (USB) memory, and the like. An operator stores a program and data on the portable recording medium 1209, and can use the program and the data by loading them into the memory 1202.

As described above, a computer-readable recording medium storing a program and data, which are used for the state determination process, includes a physical (non-transitory) recording medium such as the memory 1202, the external recording device 1205, or the portable recording medium 1209.

The network connection device 1207 is a communication interface, connected to a communication network 1210, for performing a data conversion accompanying a communication. The information processing device receives the program and the data from an external device via the network connection device 1207, and can use the program and the data by loading them into the memory 1202.

The disclosed embodiments and advantages thereof have been described in detail. A person having ordinary skill in the art could make various changes, additions, and omissions in a scope that does not depart from the present invention explicitly recited in claims.

According to embodiments of this state determination device, a state determination device that can easily determine a state of the body can be provided.

**Claims**

1. A state determination device (100, 200), comprising:

   a myopotential measurement unit (110, 202) configured to measure a myopotential of a surface of a body (150); and
   a myopotential integrated value calculation unit (120, 201) configured to calculate a myopotential integrated value by integrating, with time, the myopotential measured by the myopotential measurement unit (110, 202) for a measurement period at predetermined measurement intervals; and comprising
   a fatigue determination unit (130, 201) configured to determine presence/absence of fatigue of the body (150) based on a size of the myopotential integrated value calculated by the myopotential integrated value calculation unit (120, 201), and a change amount with time of the myopotential integrated value.

2. The state determination device (100, 200) according to claim 1, further comprising
   an aerobic exercise determination unit (201) configured to determine whether or not the body is doing an aerobic exercise based on presence/absence of a change of the myopotential integrated value calculated by the myopotential integrated value calculation unit (120, 201).

3. The state determination device (100, 200) according to claim 1, wherein
   the fatigue determination unit (130, 201) is configured to determine the presence/absence of the fatigue of the body (150) based on a change amount of a first myopotential integrated value calculated by the myopotential integrated value calculation unit (120, 201) based on the myopotential measured by the myopotential measurement unit (110, 202) for a first measurement period from a second myopotential integrated value calculated by the myopotential integrated value calculation unit (120, 201) based on a myopotential measured by the myopotential measurement unit (110, 202) for a second measurement period, which is a measurement period immediately preceding the first measurement period, and based on a sum of the first myopotential integrated value and the second myopotential integrated value.

4. The state determination device (100, 200) according to claim 3, wherein
   the fatigue determination unit (130, 201) is configured to determine that the body (150) is fatigued when the change amount of the first myopotential integrated value from the second myopotential integrated value is equal to or smaller than a particular threshold value, and the sum of the first myopotential integrated value and the second myopotential integrated value is equal to or smaller than a particular threshold value.

5. The state determination device (100, 200) according to claim 2, wherein
   the aerobic exercise determination unit (201) is configured to determine whether or not the body (150) is doing an aerobic exercise based on presence/absence of a change amount of a first myopotential integrated value calculated by the myopotential integrated value calculation unit (120, 201) based on the myopotential measured by the myopotential measurement unit (110, 202) for a first measurement period from a second myopotential integrated value calculated by the myopotential integrated value calculation unit based on the myopotential calculated by the myopotential calculation unit for a second measurement period, which is a measurement period immediately preceding the first measurement period.

6. The state determination device (100, 200) according to claim 5, wherein
   the aerobic exercise determination unit (201) is configured to determine that the body (150) is doing an aerobic exercise when the change amount of the first myopotential integrated value from the second myopotential integrated value is larger than 0.

7. The state determination device (100, 200) according to claim 2, further comprising:

an exercise intensity measurement unit (203) configured to measure an exercise intensity of the body (150); and
an exercise intensity calculation unit (201, 204) configured to calculate an exercise intensity for the measurement period by calculating an average value of the exercise intensity measured by the exercise intensity measurement unit (203) for the measurement period at the measurement intervals, wherein

the aerobic exercise determination unit (201) stores, in a storage unit (206), the exercise intensity calculated by the exercise intensity calculation unit when the aerobic exercise determination unit determines that the body (150) is doing an aerobic exercise, and estimates a minimum exercise intensity among exercise intensities stored in the storage unit as an aerobic exercise load limit, which is a lower limit of the exercise intensity with which the aerobic exercise can be done.

8. A state determination method for determining a state of a body, comprising:

measuring a myopotential of a surface of the body (150) ; and
calculating a myopotential integrated value by integrating, with time, the myopotential measured for a measurement period at predetermined measurement intervals; and further comprising
determining presence/absence of fatigue of the body (150) based on a size of the calculated myopotential integrated value, and the change amount with time of the myopotential integrated value.

9. The state determination method according to claim 8, further comprising
determining whether or not the body (150) is doing an aerobic exercise based on presence/absence of a change of the calculated myopotential integrated value.

10. A computer program adapted to cause an information processing device to execute a determination process for determining a state of a body, the determination process comprising:

measuring a myopotential of a surface of the body (150) ; and
calculating a myopotential integrated value by integrating, with time, the myopotential measured for a measurement period at predetermined measurement intervals; and further comprising
determining presence/absence of fatigue of the body (150) based on a size of the calculated myopotential integrated value, and a change amount with time of the myopotential integrated value.

11. The program according to claim 10, the determination process further comprising
determining whether or not the body (150) is doing an aerobic exercise based on presence/absence of a change of the calculated myopotential integrated value.

**Patentansprüche**

1. Zustandsfestellungsvorrichtung (100, 200), umfassend:

eine Myopotenzialmesseinheit (110, 202), die konfiguriert ist, ein Myopotenzial einer Fläche eines Körpers (150) zu messen; und
eine Myopotenzial-Integralwertberechnungseinheit (120, 201), die konfiguriert ist, einen Myopotenzialintegralwert durch Integrieren im Laufe der Zeit des Myopotenzials, das von der Myopotenzialmesseinheit (110, 202) für einen Messzeitraum
mit vorbestimmten Messintervallen gemessen wurde, zu berechnen; und umfassend
eine Müdigkeitsfeststellungseinheit (130, 201), die konfiguriert ist, das Vorhandensein/die Abwesenheit von Müdigkeit des Körpers (150) auf Grundlage einer Größe des Myopotenzialintegralwerts, der von der Myopotenzial-Integralwertberechnungseinheit (120, 201) berechnet wurde, und eines Veränderungsbetrags im Laufe der Zeit des Myopotenzialintegralwerts festzustellen.

2. Zustandsfeststellungsvorrichtung (100, 200) nach Anspruch 1, ferner umfassend
eine aerobe Übung Feststellungseinheit (201), die konfiguriert ist, auf Grundlage des Vorhandenseins/der Abwesenheit einer Veränderung des Myopotenzialintegralwerts, der von der Myopotenzial-Integralwertberechnungseinheit (120, 201) berechnet wird, festzustellen, ob der Körper eine aerobe Übung ausführt oder nicht.

3. Zustandsfeststellungsvorrichtung (100, 200) nach Anspruch 1, wobei
die Müdigkeitsfeststellungseinheit (130, 201) konfiguriert ist, das Vorhandensein/die Abwesenheit der Müdigkeit des Körpers (150) auf Grundlage einer Veränderung des Betrags eines ersten Myopotenzialintegralwerts, der von der Myopotenzial-Integralwertberechnungseinheit (120, 201) auf Grundlage des von der Myopotenzialmesseinheit (110, 202) für einen ersten Messzeitraum von einem zweiten Myopotenzialintegralwert, der von der Myopotenzial-Integralwertberechnungseinheit (120, 201) auf Grundlage eines Myopotenzials, das von der Myopotenzialmesseinheit (110, 202) für einen zweiten Messzeitraum gemessen wurde, der ein Messzeitraum ist, der dem ersten Messzeitraum unmittelbar vorausgeht, berechnet wurde, und auf Grundlage einer Summe des ersten Myopotenzialintegralwerts und des zweiten Myopotenzialintegralwerts, festzustellen.

4. Zustandsfeststellungsvorrichtung (100, 200) nach Anspruch 3, wobei
die Müdigkeitsfeststellungseinheit (130, 201) konfiguriert ist, festzustellen, dass der Körper (150) ermüdet ist, wenn der Veränderungsbetrag des ersten Myopotenzialintegralwerts von dem zweiten Myopotenzialintegralwert kleiner als oder gleich einem bestimmten Schwellenwert ist, und die Summe des ersten Myopotenzialintegralwerts und des zweiten Myopotenzialintegralwerts kleiner als oder gleich einem bestimmten Schwellenwert ist.

5. Zustandsfeststellungsvorrichtung (100, 200) nach Anspruch 2, wobei
die aerobe Übung Feststellungseinheit (201) konfiguriert ist, festzustellen, ob der Körper (150) eine aerobe Übung ausführt oder nicht, auf Grundlage des Vorhandenseins/der Abwesenheit eines Veränderungsbetrags eines ersten Myopotenzialintegralwerts, der von der Myopotenzial-Integralwertberechnungseinheit (120, 201) auf Grundlage des von der Myopotenzialmesseinheit (110, 202) für einen ersten Messzeitraum berechnet wird, von einem zweiten Myopotenzialintegralwert, der von einer der Myopotenzial-Integralwertberechnungseinheit auf Grundlage des von der Myopotenzial-Integralwertberechnungseinheit für einen zweiten Messzeitraum, der ein Messzeitraum ist, der dem ersten Messzeitraum unmittelbar vorausgeht, berechnet wird.

6. Zustandsfeststellungsvorrichtung (100, 200) nach Anspruch 5, wobei
die aerobe Übung Feststellungseinheit (201) konfiguriert ist, festzustellen, dass der Körper (150) eine aerobe Übung ausführt, wenn der Veränderungsbetrag des ersten Myopotenzialintegralwerts von dem zweiten Myopotenzialintegralwert größer als 0 ist.

7. Zustandsfeststellungsvorrichtung (100, 200) nach Anspruch 2, ferner umfassend:

eine Übungsintensitäts-Messeinheit (203), die konfiguriert ist, eine Übungsintensität des Körpers (150) zu messen; und
eine Übungsintensitäts-Berechnungseinheit (201, 204), die konfiguriert ist, eine Übungsintensität für den Messzeitraum durch Berechnen eines Durchschnittswerts der Übungsintensität, die von der Übungsintensitäts-Messeinheit (203) für den Messzeitraum zu den Messintervallen zu berechnen, wobei
die aerobe Übung Feststellungseinheit (201) die von der Übungsintensitäts-Berechnungseinheit berechnete Übungsintensität in einer Speichereinheit (206) speichert, wenn die aerobe Übung Feststellungseinheit feststellt, dass der Körper (150) eine aerobe Übung ausführt, und eine Minimum-Übungsintensität unter den Übungsintensitäten, die in der Speichereinheit gespeichert sind, als ein aerobe Übung Belastungslimit, das ein niedrigeres Limit der Übungsintensität ist, mit der die aerobe Übung ausgeführt werden kann, schätzt.

8. Zustandsfeststellungsverfahren zum Feststellen eines Zustands eines Körper, umfassend:

Messen eines Myopotenzials einer Fläche des Körpers (150); und
Berechnen eines Myopotenzialintegralwerts durch Integrieren im Laufe der Zeit des für einen Messzeitraum in vorbestimmten Messintervallen gemessenen Myopotenzials; und ferner umfassend
Feststellen des Vorhandenseins/der Abwesenheit von Müdigkeit des Körpers (150) auf Grundlage einer Größe des berechneten Myopotenzialintegralwerts und des Veränderungsbetrags im Laufe der Zeit des Myopotenzialintegralwerts.

9. Zustandsfeststellungsverfahren nach Anspruch 8, ferner umfassend
Feststellen, ob der Körper (150) eine aerobe Übung ausführt oder nicht, auf Grundlage des Vorhandenseins/der Abwesenheit einer Veränderung des berechneten Myopotenzialintegralwerts.

10. Computerprogramm, das dafür ausgelegt ist, eine informationsverarbeitende Vorrichtung zu veranlassen, einen Feststellungsprozess zum Feststellen eines Zustands eines Körpers auszuführen, wobei der Feststellungsprozess umfasst:

Messen eines Myopotenzials einer Fläche des Körpers (150); und

Berechnen eines Myopotenzialintegralwerts durch Integrieren im Laufe der Zeit des für einen Messzeitraum in vorbestimmten Messintervallen gemessenen Myopotenzials; und ferner umfassend

Feststellen des Vorhandenseins/der Abwesenheit von Müdigkeit des Körpers (150) auf Grundlage einer Größe des berechneten Myopotenzialintegralwerts und eines Veränderungsbetrags im Laufe der Zeit des Myopotenzialintegralwerts.

**11.** Programm nach Anspruch 10, wobei der Feststellungsprozess ferner umfasst:

Feststellen, ob der Körper (150) eine aerobe Übung ausführt oder nicht, auf Grundlage des Vorhandenseins/der Abwesenheit einer Veränderung des berechneten Myopotenzialintegralwerts.

**Revendications**

**1.** Dispositif de détermination d'état (100, 200), comprenant :

une unité de mesure de myopotentiel (110, 202) configurée pour mesurer un myopotentiel d'une surface d'un corps (150) ; et

une unité de calcul de valeur intégrée de myopotentiel (120, 201) configurée pour calculer une valeur intégrée de myopotentiel en intégrant, dans le temps, le myopotentiel mesuré par l'unité de mesure de myopotentiel (110, 202) pendant une période de mesure à des intervalles de mesure prédéterminés ; et comprenant

une unité de détermination de fatigue (130, 201) configurée pour déterminer la présence/l'absence de fatigue du corps (150) sur la base d'une taille de la valeur intégrée de myopotentiel calculée par l'unité de calcul de valeur intégrée de myopotentiel (120, 201), et d'une quantité de changement dans le temps de la valeur intégrée de myopotentiel.

**2.** Dispositif de détermination d'état (100, 200) selon la revendication 1, comprenant en outre

une unité de détermination d'exercice aérobique (201) configurée pour déterminer si, oui ou non, le corps est en train de faire un exercice aérobique sur la base de la présence/l'absence d'un changement de la valeur intégrée de myopotentiel calculée par l'unité de calcul de valeur intégrée de myopotentiel (120, 201).

**3.** Dispositif de détermination d'état (100, 200) selon la revendication 1, dans lequel

l'unité de détermination de fatigue (130, 201) est configurée pour déterminer la présence/l'absence de la fatigue du corps (150) sur la base d'une quantité de changement d'une première valeur intégrée de myopotentiel calculée par l'unité de calcul de valeur intégrée de myopotentiel (120, 201) sur la base du myopotentiel mesuré par l'unité de mesure de myopotentiel (110, 202) pendant une première période de mesure par rapport à une deuxième valeur intégrée de myopotentiel calculée par l'unité de calcul de valeur intégrée de myopotentiel (120, 201) sur la base d'un myopotentiel mesuré par l'unité de mesure de myopotentiel (110, 202) pendant une deuxième période de mesure, qui est une période de mesure qui précède immédiatement la première période de mesure, et sur la base d'une somme de la première valeur intégrée de myopotentiel et de la deuxième valeur intégrée de myopotentiel.

**4.** Dispositif de détermination d'état (100, 200) selon la revendication 3, dans lequel

l'unité de détermination de fatigue (130, 201) est configurée pour déterminer que le corps (150) est fatigué lorsque la quantité de changement de la première valeur intégrée de myopotentiel par rapport à la deuxième valeur intégrée de myopotentiel est inférieure ou égale à une valeur de seuil particulière, et que la somme de la première valeur intégrée de myopotentiel et de la deuxième valeur intégrée de myopotentiel est inférieure ou égale à une valeur de seuil particulière.

**5.** Dispositif de détermination d'état (100, 200) selon la revendication 2, dans lequel

l'unité de détermination d'exercice aérobique (201) est configurée pour déterminer si, oui ou non, le corps (150) est en train de faire un exercice aérobique sur la base de la présence/l'absence d'une quantité de changement d'une première valeur intégrée de myopotentiel calculée par l'unité de calcul de valeur intégrée de myopotentiel (120, 201) sur la base du myopotentiel mesuré par l'unité de mesure de myopotentiel (110, 202) pendant une première période de mesure par rapport à une deuxième valeur intégrée de myopotentiel calculée par l'unité de calcul de valeur intégrée de myopotentiel sur la base du myopotentiel calculé par l'unité de calcul de myopotentiel pendant une deuxième période de mesure, qui est une période de mesure qui précède immédiatement la première période de mesure.

**6.** Dispositif de détermination d'état (100, 200) selon la revendication 5, dans lequel l'unité de détermination d'exercice aérobique (201) est configurée pour déterminer que le corps (150) est en train de faire un exercice aérobique lorsque la quantité de changement de la première valeur intégrée de myopotentiel par rapport à la deuxième valeur intégrée de myopotentiel est supérieure à 0.

**7.** Dispositif de détermination d'état (100, 200) selon la revendication 2, comprenant en outre :

une unité de mesure d'intensité d'exercice (203) configurée pour mesurer une intensité d'exercice du corps (150) ; et

une unité de calcul d'intensité d'exercice (201, 204) configurée pour calculer une intensité d'exercice pendant la période de mesure en calculant une valeur moyenne de l'intensité d'exercice mesurée par l'unité de mesure d'intensité d'exercice (203) pendant la période de mesure à des intervalles de mesure, dans lequel l'unité de détermination d'exercice aérobique (201) mémorise, dans une unité de mémorisation (206), l'intensité d'exercice calculée par l'unité de calcul d'intensité d'exercice lorsque l'unité de détermination d'exercice aérobique détermine que le corps (150) est en train de faire un exercice aérobique, et estime une intensité d'exercice minimum parmi les intensités d'exercice mémorisées dans l'unité de mémorisation en tant que limite de charge d'exercice aérobique, qui est une limite inférieure de l'intensité d'exercice avec laquelle l'exercice aérobique peut être fait.

**8.** Procédé de détermination d'état pour déterminer un état d'un corps, comprenant :

la mesure d'un myopotentiel d'une surface du corps (150) ; et

le calcul d'une valeur intégrée de myopotentiel en intégrant, dans le temps, le myopotentiel mesuré pendant une période de mesure à des intervalles de mesure prédéterminés ; et comprenant en outre

la détermination de la présence/l'absence de fatigue du corps (150) sur la base d'une taille de la valeur intégrée de myopotentiel calculée, et de la quantité de changement dans le temps de la valeur intégrée de myopotentiel.

**9.** Procédé de détermination d'état selon la revendication 8, comprenant en outre la détermination si, oui ou non, le corps (150) est en train de faire un exercice aérobique sur la base de la présence/l'absence d'un changement de la valeur intégrée de myopotentiel calculée.

**10.** Programme d'ordinateur conçu pour amener un dispositif de traitement d'informations à exécuter un processus de détermination pour déterminer un état d'un corps, le processus de détermination comprenant :

la mesure d'un myopotentiel d'une surface du corps (150) ; et

le calcul d'une valeur intégrée de myopotentiel en intégrant, dans le temps, le myopotentiel mesuré pendant une période de mesure à des intervalles de mesure prédéterminés ; et comprenant en outre

la détermination de la présence/l'absence de fatigue du corps (150) sur la base d'une taille de la valeur intégrée de myopotentiel calculée, et d'une quantité de changement dans le temps de la valeur intégrée de myopotentiel.

**11.** Programme selon la revendication 10, le processus de détermination comprenant en outre la détermination si, oui ou non, le corps (150) est en train de faire un exercice aérobique sur la base de la présence/l'absence d'un changement de la valeur intégrée de myopotentiel calculée.

100

110    150

MYOPOTENTIAL
MEASUREMENT UNIT

120

MYOPOTENTIAL
INTEGRATED VALUE
CALCULATION UNIT

130

FATIGUE DETERMINATION
UNIT

F I G. 1

F I G. 2

F I G. 3

START ~ S400

A

MEASURING MYOPOTENTIAL ~ S401

MEASURING EXERCISE INTENSITY ~ S402

S403
ONE MINUTE ELAPSED? — NO

YES

S404
STOP REQUEST ISSUED? — YES

NO

S419

END

CALCULATING MYOPOTENTIAL INTEGRATED VALUE BY INTEGRATING MYOPOTENTIAL MEASURED FOR 1 MINUTE WITH TIME ~ S405

CALCULATING SUM OF MYOPOTENTIAL INTEGRATED VALUE CALCULATED AT THIS TIME (FIRST MYOPOTENTIAL INTEGRATED VALUE) AND MYOPOTENTIAL INTEGRATED VALUE CALCULATED AT PRECEDING TIME (SECOND MYOPOTENTIAL INTEGRATED VALUE) ~ S406

CALCULATING CHANGE AMOUNT D1 OF FIRST MYOPOTENTIAL INTEGRATED VALUE FROM SECOND MYOPOTENTIAL INTEGRATED VALUE ~ S407

CALCULATING AVERAGE VALUE OF EXERCISE INTENSITY MEASURED FOR 1 MINUTE ~ S408

CALCULATING CHANGE AMOUNT D2 OF AVERAGE VALUE OF EXERCISE INTENSITY CALCULATED AT THIS TIME (FIRST EXERCISE INTENSITY) FROM AVERAGE VALUE OF EXERCISE INTENSITY CALCULATED AT PRECEDING TIME (SECOND EXERCISE INTENSITY) ~ S409

B

F I G. 4

F I G. 5

F I G. 6

F I G. 7

YOU ARE CURRENTLY DOING
AEROBIC EXERCISE WITH
3.4 [Mets·h] (100 STEPS
PER MINUTE)

YOU CAN DO AEROBIC
EXERCISE BY CONTINUING
EXERCISE WITH CONSTANT
EXERCISE INTENSITY OF
3.4 [Mets·h] OR HIGHER
(100 STEPS PER MINUTE)

F I G. 8

YOUR BODY IS FATIGUED AT
AND AFTER 20:32

FIG. 9

F I G. 1 0

F I G. 1 1

BUS

F I G. 1 2

**REFERENCES CITED IN THE DESCRIPTION**

**Patent documents cited in the description**

- JP 2007075172 A
- JP 2007209453 A
- WO 1999043392 A
- US 20110118621 A